# EUROPÄISCHE PATENTSCHRIFT

(11) **EP 1 438 924 B1**
(45) Veröffentlichungstag und Bekanntmachung des Hinweises auf die Patenterteilung: **12.03.2008**
(21) Anmeldenummer: 03016384.4
(22) Anmeldetag: 19.07.2003
(51) Int. Cl.: A61B 19/02

(54) **Abfallbehälter**
Waste container
Poubelle

(30) Priorität: 08.08.2002 DE 10236378
(43) Veröffentlichungstag der Anmeldung: 21.07.2004
(73) Patentinhaber: Udo Heisig GmbH, 85640 Putzbrunn (DE)
(72) Erfinder: Heisig, Udo, 85640 Putzbrunn (DE)
(74) Vertreter: Feldkamp, Rainer

(56) Entgegenhaltungen:
- GB-A- 1 600 917
- US-A- 4 625 877
- US-A- 4 883 173
- US-A- 5 323 902
- US-A- 5 402 887

## Beschreibung

Die vorliegende Erfindung betrifft einen Abfallbehälter der im Oberbegriff des Patentanspruchs 1 genannten Art.

Derartige Abfallbehälter, die beispielsweise aus der EP 0 343 680 B1 bekannt sind, werden insbesondere im medizinischen Bereich verwendet, um medizinische Abfälle, insbesondere spitzes oder scharfkantiges Abfallgut wie Injektionsspritzen, Kanülen, Skalpelle und Glasbruch, aber auch gebrauchtes Verbandsmaterial oder sonstige Abfälle zu entsorgen, die beispielsweise durch Viren oder Bakterien kontaminiert sein können.

Bei derartigen Abfallbehältern ist es erforderlich, daß der Einfüllöffnungs-Verschlußdeckel vor der Entsorgung des Abfallbehälters endgültig derart verschlossen wird, daß er zumindest ohne Gewaltanwendung nicht mehr geöffnet werden kann. Diese Anforderung ergibt sich daraus, daß ein Verletzungs- oder sogar Infektionsrisiko durch in dem Abfallbehälter enthaltene kontaminierte Abfälle für die Personen vermieden werden soll, die vor oder während der Entsorgung mit dem Abfallbehälter hantieren. Der Verschlußdeckel kann entweder die gesamte Öffnung des Abfallbehälterkörpers verschließen, doch wird diese Öffnung zumeist durch einen Abfallbehälterdeckel verschlossen, in dem dann eine Einfüllöffnung ausgebildet ist, die durch einen getrennten kleineren Verschlußdeckel verschließbar ist.

Ein Teil der Einfüllöffnung ist segmentförmig unterhalb des Verschlußdeckels durch eine Abstreifplatte oder einen Steg abgedeckt, die mit Abstreifkerben oder Abstreiföffnungen versehen ist, die zum Abziehen von Kanülen von Spritzen dienen. Bei einigen Kanülen ist zur Trennung von der Spritze eine Drehung zwischen Kanüle und Spritze erforderlich, was voraussetzt, daß die Kanüle in der Abstreifkerbe oder Abstreiföffnung zumindest vorübergehend gegen eine Drehung festgehalten werden kann, wie dies beispielsweise in der US-A-5 402 887 beschrieben ist. Hierbei ergibt sich das Problem, das die Kanüle nach der Trennung von der Spritze in der Abstreifkerbe oder Abstreiföffnung hängenbleibt.

Der vorliegenden Erfindung liegt die Aufgabe zugrunde, einen Abfallbehälter der eingangs genannten Art dahingehend weitertzubilden, daß die Kanülen leicht und gefahrlos aus der Abstreifkerbe oder Abstreiföffnung entfembar sind.

Diese Aufgabe wird durch die im kennzeichnenden Teil des Patentanspruchs 1 angegebenen Merkmale gelöst.

Vorteilhafte Weiterbildungen und Ausgestaltungen der Erfindung ergeben sich aus den Unteransprüchen.

Der erfindungsgemäße Abfallbehälter ermöglicht ein Entfernen einer gegenenfalls in der Abstreifkerbe oder Abstreiföffnung festhängenden Kanüle durch leichtes Schließen des Verschlußdeckels der Einfüllöffnung, wobei der oder die Stößel an der Unterseite des Verschlußdeckels in die Abstreifkerbe oder Abstreiföffnung eindringen und darin festhängende Gegenstände in das Innere des Abfallbehälters stoßen. Hierbei wird eine Verletzungsgefahr ausgeschlossen

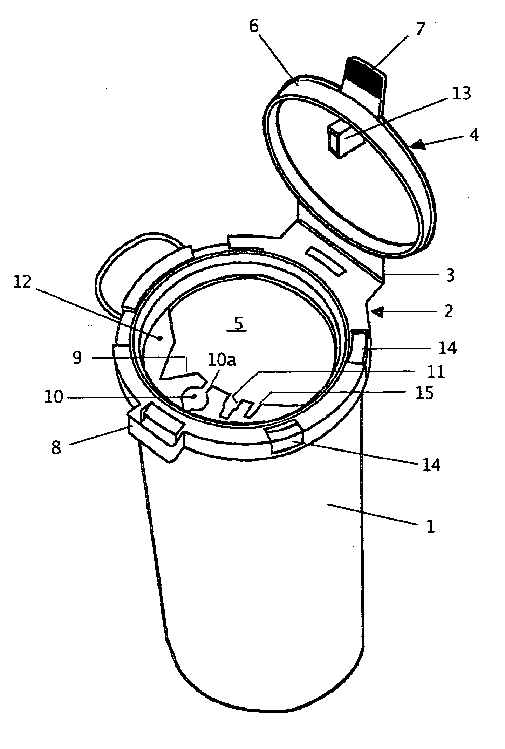

Ausführungsbeispiele der Erfindung werden im folgenden anhand der Zeichnung noch näher erläutert.

In der Zeichnung ist eine perspektivische Ansicht einer Ausführungsform eines Abfallbehälters mit einem Abfallbehälterdeckel gezeigt.

Der Abfallbehälter 1 und der Abfallbehälterdeckel 2 können beispielsweise gemäß der EP 0 343 680 über Rasteinrichtungen miteinander verbunden sein. Zu diesem Zweck weist der Abfallbehälterdeckel 2 weiterhin am Umfang verteilte Rastlaschen 14 auf, die unter einen Wulst am oberen Ende des Abfallbehälters 1 greifen und damit den Abfallbehälterdeckel 2 unlösbar mit dem Abfallbehälter 1 verbinden.

Mit dem Abfallbehälterdeckel 2 ist über ein Filmscharnier 3 ein Verschlußdeckel 4 gelenkig verbunden. Der Abfallbehälterdeckel 2 weist eine Einfüllöffnung 5 auf, in der ein Steg 12 angeordnet ist, der Abstreifkerben und/oder Abstreiföffnungen, beispielsweise zum Abstreifen der Kanülen von Spritzen und dergleichen aufweist.

Der Verschlußdeckel 4 weist einen ringförmigen Flansch 6 auf, dessen Außendurchmesser an den Innendurchmesser der Einfüllöffnung 5 angepaßt ist und der in diese Öffnung mit leichtem Haftsitz eindrückbar ist, wobei der Verschlußdeckel 4 wieder aus dieser Einfüllöffnung herausgezogen werden kann.

Der Verschlußdeckel 4 weist weiterhin eine Lasche 7 auf, die mit dem Verschlußdeckel 4 über ein Filmscharnier verbunden ist, so daß der außenliegende Teil der Lasche 7 gegenüber dem Verschlußdeckel senkrecht zur Hauptebene des Verschlußdeckels 4 abklappbar ist.

Der Abfallbehälterdeckel 2 ist mit einer Verschlußhülse 8 versehen, die sich in vorteilhafter Weise am Außenumfang des Abfallbehälterdeckels 2 befindet, jedoch auch in einem Innenbereich des Abfallbehälterdeckels und damit nach dessen Aufsetzen auf den Abfallbehälterkörper im Inneren dieses Abfallbehälterkörpers liegen kann.

Die Verschlußhülse 8 weist in ihrem Inneren einen Aufnahmekanal für das freie Ende der Lasche 7 auf, dessen Längsachse sich im wesentlichen senkrecht zur Hauptebene des Abfallbehälterdeckels 2 erstreckt.

Wenn das freie Ende der Lasche 7 um das Filmscharnier herum senkrecht abgewinkelt ist, so kann dieses freie Ende in den Aufnahmekanal der Verschlußhülse 8 eingeführt werden, und im Inneren der Verschlußhülse 8 angeordnete Rasteinrichtungen wirken dann mit auf der Lasche ausgebildeten Rasteinrichtungen zusammen, um diese Lasche 7 sicher und unlösbar in der Verschlußhülse 8 zu verriegeln, so daß ein Öffnen des Verschlußdeckels in dieser Schließstellung nur noch mit extremer Gewalt möglich ist.

Der Steg 12 weist Abstreifkerben oder Abstreiföffnungen 9, 10, 11, 15 auf, die zum Abstreifen von Kanülen und dergleichen von Spritzen oder anderen medizinischen Instrumenten dienen, damit die Trennung beispielsweise der Kanüle und deren Entsorgung ohne Gefahr für den Benutzer möglich ist.

Bei manchen Kanülen ist zur Trennung der Kanüle von der Spritze oder einem anderen medizinischen Instrument eine Drehung zwischen der Spritze und der Kanüle erforderlich, so daß das der Spritze benachbarte Ende der Kanüle an zumindest zwei diametral gegenüberliegenden Bereichen der Innenkanten der Abstreifkerbe oder Abstreiföffnung erfaßt werden sollte, wie dies in der US-A-5 402 887 erläutert ist.

Eine derartige Drehung ist beispielsweise mit den Abstreifkerben oder -öffnungen 10, 11, 15 möglich. Hierbei ergibt sich jedoch das Problem, daß die Abstreifkerbe oder Abstreiföffnung einerseits die Kanüle so gut festhalten sollte, daß das Abdrehen der Kanüle von der Spritze möglich ist, daß dann jedoch andererseits die Kanüle in der Abstreifkerbe oder Abstreiföffnung festhängt und nicht in das Innere des Abfallbehälters fällt.

Um eine Entfernung einer Kanüle aus der Abstreifkerbe oder Abstreiföffnung zu ermöglichen, ohne daß die Gefahr besteht, daß der Benutzer mit der Kanüle in Berührung kommt, ist erfindungsgemäß an der Unterseite des Verschlußdeckels 4 zumindest ein Stößel 13 vorgesehen, der an einer derartigen Stelle angeordnet ist, daß er beim Schließen des Verschlußdeckels 4 durch die Abstreifkerbe oder Abstreiföffnung 10, 11, 15 hindurch vorspringt und darin gegebenenfalls festhängende Gegenstände sicher in den Abfallbehälter ausstößt.

Der oder die Stößel 13 weisen vorzugsweise eine derartige Länge auf, daß das Ausstoßen bereits bei einer leichten Bewegung des Verschlußdeckels 4 in Richtung auf die Einfüllöffnung 5 möglich ist.

Weiterhin können der oder die Stößel 13 eine zumindest teilweise der Form der zugeordneten Abstreifkerbe oder Abstreiföffnung 10, 11, 15 entsprechende Form aufweisen, es reicht jedoch aus, wenn der Stößel derartige Abmessungen aufweist, daß er in jedem Fall mit einem in der Abstreifkerbe oder Abstreiföffnung festhängenden Gegenstand in Eingriff kommt und diesen auswirft.

Der oder die Stößel 13 können vorzugsweise einstückig mit dem Behälterdeckel 4 ausgebildet sein, oder sie können als getrennte Elemente and dem Behälterdeckel befestigt werden.

Damit die Abstreifkerbe oder Abstreiföffnung einerseits die Kanüle so gut festhalten sollte, daß das Abdrehen der Kanüle von der Spritze möglich ist, und daß andererseits die Kanüle aus einer Abstreiföffnung mit Hilfe des Stößels leicht auswerfbar ist, wird diese Abstreiföffnung vorzugsweise nicht mit einem geschlossenen Umfang ausgebildet, sondern der Steg 12 weist ausgehend von der Abstreiföffnung einen Schlitz auf, wie dies für die Abstreiföffnung 10 gezeigt ist. Dieser Schlitz 10a verbindet die Abstreiföffnung 10 mit der Einfüllöffnung 5. Ein weiterer Schlitz kann sich diametral gegenüberliegend zum Schlitz 10a erstrecken, so daß die Wände der Abstreiföffnung 10 zwei gegenüberliegende Klemmbacken bilden, die die Kanüle beim Abdrehen sicher festhalten, jedoch ein leichtes Ausstoßen der Kanüle durch den Stößel 13 ermöglichen

Derartige Schlitze 10a erleichtern weiterhin eine bogenförmige Bewegung des Stößels 13 beim Schließen des Verschlußdeckels 4.

## Patentansprüche

1. Abfallbehälter, insbesondere für medizinische Abfälle, mit einer Einfüllöffnung (5), die durch einen Verschlußdeckel (4) verschließbar ist, der mit dem Abfallbehälter beweglich verbunden ist, wobei ein Teil der Einfüllöffnung (5) durch einen Steg (12) abgedeckt ist, der Abstreifkerben und/oder Abstreiföffnungen (9, 10, 11, 15) aufweist, wobei der Verschlußdeckel (2) einen sich in Richtung auf das Innere des Abfallbehäters erstreckenden Stößel (13) aufweist
**dadurch gekennzeichnet, daß** der Stößel (13) in Verschlußstellung des Verschlußdeckels (2) in einer den Abstreifkerben und/oder Abstreiföffnungen (9, 10, 11, 15) gegenüberliegenden Position angeordnet ist, und der Stößel (13) derart geformt ist, daß er beim Schließen des Verschlußdeckels (4) die zugeordnete Abstreifkerbe und/oder Abstreiföffnung (9, 10, 11, 15) durchdringt und darin befindliche Gegenstände in das Innere des Abfallbehälters ausstößt.

2. Abfallbehälter nach Anspruch 1,
**dadurch gekennzeichnet, daß** der Querschnitt des Stößels (13) zumindest teilweise der Form der Abstreifkerben und/oder Abstreiföffnungen (9, 10, 11, 15) entspricht.

3. Abfallbehälter nach Anspruch 1 oder 2,
**dadurch gekennzeichnet, daß** die Länge des Stößels (13) so gewählt ist, daß das Ausstoßen der Gegenstände bereits bei einem teilweisen Schließen des Verschlußdeckels (4) beginnt.

4. Abfallbehälter nach einem der vorhergehenden Ansprüche,
**dadurch gekennzeichnet, daß** der Stößel (13) einstückig mit dem Verschlußdeckel (4) ausgebildet ist.

5. Abfallbehälter nach einem der vorhergehenden Ansprüche,
**dadurch gekennzeichnet, daß** der Verschlußdeckel (4) mit dem Abfallbehälter über eine Gelenkverbindung (3) beweglich verbunden ist.

6. Abfallbehälter nach einem der vorhergehenden Ansprüche,
**dadurch gekennzeichnet, daß** zumindest eine der Abstreiföffnungen (10) im wesentlichen kreisförmig ausgebildet ist, und daß sich zumindest ein Schlitz (10a) von der Abstreiföffnung (10) durch den Steg (12) hindurch bis zur Einfüllöffnung (5) erstreckt, um ein Aufspreizen der Abstreiföffnung (10) zu ermöglichen.

## Claims

1. A waste container, especially for medical waste, having a charging hole (5) that can be closed by a cover lid (4) movably connected to the waste container, with a part of the charging hole (5) being covered by a partition wall (12) comprising stripping slots and/or stripping openings (9, 10, 11, 15), the cover lid (2) comprising a pusher (13) extending towards the interior of the waste container,
**characterised in that** in the closed position of the cover lid (2) the pusher (13) is disposed in a position opposite the stripping slots and/or stripping openings (9, 10, 11, 15) and the pusher (13) is shaped such that, when the cover lid (4) is closed, it penetrates the associated stripping slot and/or stripping opening (9, 10, 11, 15) and ejects items situated therein into the interior of the waste container.

2. A waste container according to Claim 1,
**characterised in that** the cross section of the pusher (13) corresponds at least to some extent to the shape of the stripping slots and/or stripping openings (9, 10, 11, 15).

3. A waste container according to Claim 1 or 2,
**characterised in that** the length of the pusher (13) is chosen so that the ejection of the items starts even when the cover lid (4) is partially closed.

4. A waste container according to one of the preceding Claims,
**characterised in that** the pusher (13) is constructed in one piece with the cover lid (4).

5. A waste container according to one of the preceding Claims,
**characterised in that** the cover lid (4) is flexibly connected to the waste container via a link joint (3).

6. A waste container according to one of the preceding Claims,
**characterised in that** at least one of the stripping openings (10) has a substantially circular construction,
and **in that** at least one slit (10a) extends from the stripping opening (10) through the partition wall (12) to the charging hole (5) to enable the stripping opening (10) to be forced open.

## Revendications

1. Poubelle, notamment pour des déchets médicaux, dotée d'un orifice de remplissage (5) qui peut être fermé par un couvercle (4) qui est relié de manière mobile à la poubelle, une partie de l'orifice de remplissage (5) étant recouverte par une traverse (12) qui présente des entailles de raclage et/ou des orifices de raclage (9, 10, 11, 15), le couvercle (2) présentant un coulisseau (13) s'étendant en direction de l'intérieur de la poubelle, **caractérisée en ce que** le coulisseau (13) est disposé en position de fermeture du couvercle (2) dans une position opposée aux entailles de raclage et/ou orifices de raclage (9, 10, 11, 15) et le coulisseau (13) est formé de telle sorte qu'il pénètre, lors de la fermeture du couvercle (4), l'entaille de raclage associée et/ou l'orifice de raclage (9, 10, 11, 15) et expulse des objets se trouvant dedans à l'intérieur de la poubelle.

2. Poubelle selon la revendication 1,
**caractérisée en ce que** la section du coulisseau (13) correspond au moins partiellement à la forme des entailles de raclage et/ou des orifices de raclage (9, 10, 11, 15).

3. Poubelle selon la revendication 1 ou 2,
**caractérisée en ce que** la longueur du coulisseau (13) est choisie de telle sorte que l'expulsion des objets commence dès une fermeture partielle du couvercle (4).

4. Poubelle selon l'une quelconque des revendications précédentes,
**caractérisée en ce que** le coulisseau (13) est réalisé d'un seul tenant avec le couvercle (4).

5. Poubelle selon l'une quelconque des revendications précédentes,
**caractérisée en ce que** le couvercle (4) est relié de manière mobile à la poubelle par le biais d'un assemblage articulé (3).

6. Poubelle selon l'une quelconque des revendications précédentes,
**caractérisée en ce qu'**au moins l'un des orifices de raclage (10) est réalisé essentiellement de manière circulaire, et **en ce qu'**au moins une fente (10a) s'étend de l'orifice de raclage (10), au travers de la traverse (12) jusqu'à l'orifice de remplissage (5) afin de permettre un écartement de l'orifice de raclage (10).
